# EUROPEAN PATENT SPECIFICATION

(11) **EP 4 142 854 B1**
(45) Date of publication and mention of the grant of the patent: **29.04.2026**
(21) Application number: 21726296.3
(22) Date of filing: 28.04.2021
(51) Int. Cl.: A61M 27/00, A61M 25/00

(54) **SYSTEM AND METHOD FOR A COVERING**
SYSTEM UND VERFAHREN FÜR EINE ABDECKUNG
SYSTÈME ET PROCÉDÉ POUR UN REVÊTEMENT

(30) Priority: 29.04.2020 US 202016862072
(43) Date of publication of application: 08.03.2023
(73) Proprietor: Medtronic Xomed, Inc., Jacksonville, FL 32216 (US)
(72) Inventor: PEACOCK, Bruce T., Minneapolis, Minnesota 55432- 3568 (US); NAGY, Elizabeth K., Minneapolis, Minnesota 55432- 3568 (US)
(74) Representative: Viering, Jentschura & Partner mbB Patent- und Rechtsanwälte
(86) International application number: PCT/US2021/029632
(87) International publication number: WO 2021/222400

(56) References cited:
- EP-A1- 1 491 229
- EP-A1- 2 436 419
- EP-B1- 2 436 419
- CN-A- 110 935 091
- US-A1- 2012 089 124

## Description

### FIELD

The subject disclosure relates to a covering, and particularly to a covering material for at least a portion of a catheter.

### BACKGROUND

This section provides background information related to the present disclosure which is not necessarily prior art.

A subject, such as a human patient, may have a condition or ailment for which a treatment may be described. The ailment may include hydrocephalous which generally includes an overproduction of cerebral fluid in the ventricles of the brain and/or an abnormal absorption or outflow of cerebral fluid from the brain. The condition, therefore, may cause an inappropriate or undesirable increase in volume of cerebral spinal fluid (CSF) within the ventricles in the brain and an increased pressure on the brain within the skull.

In various instances a shunt may be implanted into the subject. The shunt may include an inflow catheter positioned within a ventricle of the brain and an outflow catheter positioned at a location remote from the brain. The excess cerebral spinal fluid may, therefore, flow from the ventricle to a selected location in the subject. The flow of the CSF from the ventricle through the inflow and outflow catheters may allow for an appropriate or selected volume of CSF within the brain to achieve a selected pressure on the brain within the skull. Over a period of time, however, the inflow or outflow catheter may become obstructed with material and require revision to ensure proper or selected operation of the shunt system.

Exemplary catheters and/or shunt systems are disclosed in US 2012/089124 A1, CN 110 935 091 A, EP 2 436 419 A1, and EP 1 491 229 A1.

### SUMMARY

According to the present invention, a hydrocephalus shunt system according to claim 1 and a method of assembling a hydrocephalus shunt system according to claim 8 are provided. Further embodiments are defined in the dependent claims.

This section provides a general summary of the disclosure, and is not a comprehensive disclosure of its full scope or all of its features.

A catheter may be positioned in a selected portion of a subject, such as within a ventricle of a brain of a human subject. The catheter may include passages, such as bores, through a selected portion of a catheter. In various embodiments, a through bore may be formed through an outer wall of a catheter. The catheter, therefore, may include an elongated wall structure with one or more through bores formed therethrough.

The catheter may further include an internal cannula or passage to allow flow of a selected material, such as a liquid, therethrough. In various embodiments, the catheter may allow for flow of cerebral spinal fluid (CSF). The catheter may be implanted as a part of a shunt system to shunt or drain CSF from a first location to a second location.

Positioned over at least a portion of the catheter, such as a portion including at least a number of the through bores of the catheter, may be a selected material. The selected material may be positioned on the catheter as a sleeve, film, or other appropriate configuration. The covering material may operate as a filter or bore protecting portion to assist in or maintaining a selected through put of flow through the bores formed into the catheter. In addition, or alternatively, the covering may also operate as an ingrowth and/or encapsulation inhibitor. Thus, the covering may inhibit ingrowth and/or encapsulation in to the bores and/or onto the catheter.

Further areas of applicability will become apparent from the description provided herein. The description and specific examples in this summary are intended for purposes of illustration only and are not intended to limit the scope of the present disclosure.

### DRAWINGS

The drawings described herein are for illustrative purposes only of selected embodiments and not all possible implementations, and are not intended to limit the scope of the present disclosure.
Figs. 1A and 1B are a catheter with the filter material positioned over an inlet area, according to various embodiments;
Fig. 2 is a detailed view of an inlet area of a catheter and filter material positioned thereon;
Fig. 3 is an environmental schematic view of a shunt and system positioned in a subject, according to various embodiments;
Fig. 4A is a schematic view of an assembly of a catheter and filter portion, according to various embodiments; and
Fig. 4B is an assembly of a catheter and filter portion, according to various embodiments.

Corresponding reference numerals indicate corresponding parts throughout the several views of the drawings.

### DETAILED DESCRIPTION

Example embodiments will now be described more fully with reference to the accompanying drawings.

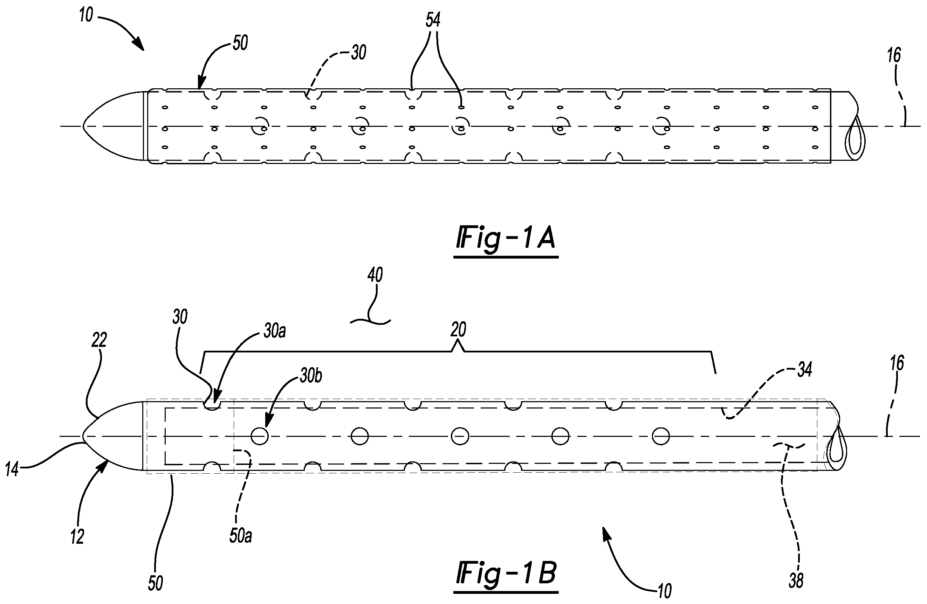

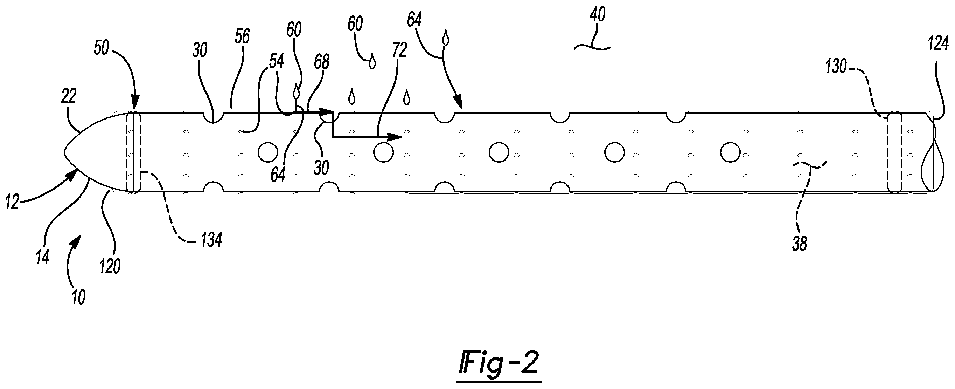

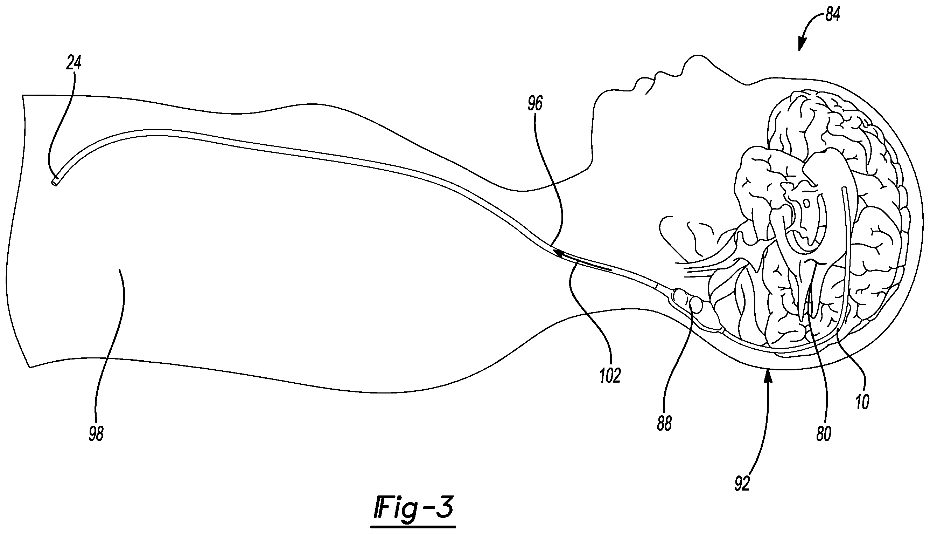

With initial reference to Figs. 1A and 1B and Fig. 2, a catheter 10 is illustrated. The catheter 10 includes a member or wall structure 12 that includes or defines an exterior surface 14. The catheter 10 may include a selected length or be formed along a long or longitudinal axis 16. In various embodiments, the catheter 10 may be provided as an inflow or inlet catheter or portion for a hydrocephalous shunt. The hydrocephalous shunt may be configured, such as formed, assembled, and/or implanted to shunt cerebral spinal fluid (CSF) from a position near a first or inlet portion 20 to a second or distal end 24 (Fig. 3) at a position remote or away from the inlet end or portion 20.

The inlet portion 20 may be substantially at or near a distal or terminal end 22 of the catheter 10. Near the inlet end or formed through the inlet portion 20 may be a plurality of through bores 30. A plurality of the through bores 30 may be formed through the external surface 14 of the wall 12 and to an internal surface 34 through the wall 12. The internal surface 34 may define or form a passage 38. The passage 38 may be formed through the catheter 10 from the terminal end 22, or near the terminal end 22, through the catheter 10. The passage 38 may be formed as a cannula within the catheter 10. In various embodiments, as discussed further herein, the catheter 10 may be an inlet catheter and may be connected to a selected portion, as discussed further herein.

The one or more of the inlet passages 30 of the inlet portion 20 may allow a material to pass through the wall 12. The material may move from an external environment 40 through the passages 30 of the external catheter wall 12 to an internal environment in the passage 38. Thus, material may move into the catheter 10 from exterior to the catheter 10.

The inlet portion 20 of the catheter may be formed of one or more layers and the bores 30 formed entirely therethrough. The passages 30, therefore, may be covered or protected by a covering or overlaying member 50. The overlaying member 50 may be applied or assembled onto the catheter 10 after the bores 30 are formed through the wall 12.

The catheter 10 may, therefore, be at least partially covered or encapsulated with the overlaying member 50. The overlaying member 50 may also be referred to as a filter 50. The filter 50 may include a material of a selected thickness (a distance between an external surface in contact with the environment 40 and an internal surface nearer (e.g. in contact with) the catheter), such as about 0.001 millimeters (mm) to about 0.1 mm, and further including about 0.004 mm to about 0.8 mm, and further including about 0.004 mm to about 0.009mm. The filter 50 may be formed in any appropriate manner, such as being formed as a tube, as discussed herein, to be moved and/or positioned on the catheter 10, such as over an exterior surface 14 of the wall 12. The filter 50 may, however, also be formed as an elongated portion, as discussed herein, that may be wrapped around the catheter 10, such as in a helical manner, to extend along a length of the catheter 10. In various embodiments, the filter 50 is configured to cover at least a portion, including all of, the inlet area 20. Thus, the filter may be configured and assembled to cover all of the bores 30 of the catheter 10.

The filter 50 may be formed of appropriate materials such as those including polytetrafluoroethylene (PTFE). In various embodiments, the material may be an expanded polytetrafluoroethylene. The filter material may be selected or formed to allow a first selected material to pass through and into the bores 30 and inhibit or stop a second selected material from passing into the bores 30. Exemplary materials include the AEOS^{®}, FILTRIQ^{®} and/or BioWeb^{™} materials, both sold by Zeus, Inc.

The filter 50 includes one or more through bores or via's 54 formed through a wall or surface 56 of the filter 50. The through bore 54 may allow for a selected material, such as a fluid, including CSF, to pass through the through bore 54. The through bore 54, however, inhibits or stops a second material, such as tissue, from passing through the filter 50.

With specific reference to Fig. 2, schematically illustrated is a fluid 60 which may move or surround the filter 50 in the external environment 40. At a selected rate, the fluid 60 may move through the bore 54 generally in the direction of arrow 64. The fluid 60 may then be encased and/or flow between the filter 50 and the external surface 14 of the catheter 10. As discussed above, the catheter 10, such as in the inlet area 20, includes one or more through bores 30. The fluid 60, therefore, may travel toward and through one or more of the through bores 54 generally in the direction of arrow 64. The fluid may flow generally in the direction of arrow 68 to pass through the through hole 30 and into the interior passage 38 of the catheter 10. Within the passage 38, the fluid may then generally flow and may travel in a selected direction, such as generally in the direction of arrow 72.

With continuing reference to Figs. 1A, 1B, and 2, and additional reference to Fig. 3, the fluid 60 may flow along the catheter 10, which may be an inlet catheter 10 from a ventricle 80 in a subject 84. As is generally understood by one skilled in the art, the inlet catheter 10 may be positioned (i.e. implanted) in the ventricle to allow the fluid 60 to be drained away from the ventricle 80. The inlet catheter 10 may be a part of a shunt system that includes a selected flow control system, such as a valve 88.

The valve 88 may be implanted in the subject 84 in an appropriate position. In various embodiments, the valve 88 may be implanted generally near a skull 92 of the subject 84, torso of the subject 84, or any other appropriate location. It is understood that the inlet catheter 10 may be connected to the valve 88, or any other appropriate flow control mechanism or system, such as a pump, adjustable valve, fixed valve, or the like. The inlet catheter 10 and the valve 88 may both be implanted in the subject 84.

The valve 88 may be further connected to an outlet catheter 96. The outlet catheter 96 may extend from the valve 88 to a selected location, such as a peritoneal cavity 98 of the subject 84. The inlet catheter 10, the valve 88, and the outlet catheter 96 may generally be understood to be a shunt system, such as a hydrocephalus shunt system. The shunt system may be entirely implanted in the subject 84.

The fluid may flow in the direction of arrow 72 through the inlet catheter 10, through the valve 88, and through the outlet catheter 96 generally in the direction of arrow 102. The fluid may then drain or pass through the outlet catheter 96 into a peritoneal cavity 98, or any other appropriate location, of the subject 84. It is understood that the outlet catheter 96 may be positioned within the subject 84 in an appropriate location to allow for draining of the CSF from the ventricle 80 to an appropriate location, such as one with high blood flow. Accordingly, as illustrated in Fig. 3, the inlet catheter 10, the valve 88 and the outlet catheter 96 may be implanted or positioned in the subject 84 as a CSF shunt system.

When positioned in the subject 84, the filter 50 may limit entirely and/or substantially limit material coming into or moving into contact with the inlet area 20, including the through bores 30, to the fluid 60. In other words, the filter 50 may inhibit non-fluid from passing or moving into the passages 30 of the catheter 10. The filter 50, therefore, provides a physical barrier between the through bores 30 and an external environment 40. The inlet catheter 10 that may be positioned and fixed within the subject 84 may be positioned in an anatomical location. Accordingly, the filter 50 may limit or eliminate solid or soft tissue ingrowth past the filter 50 toward the catheter 10. Accordingly, the through bores 30 may remain substantially open, even over a period of time, such as including 5 years, greater than 10 years, greater than 20 years, or an appropriate period. Thus, the filter 50 may extend a useful life of at least the inlet catheter 10 and/or the entire shunt system.

The filter 50, therefore, allows the fluid 60 to pass through the filter 50 and through the through bores 30 into the catheter 10. Soft tissue or other tissue or particles, however, having a diameter greater than the size of the through bore 54 in the filter 50, may not pass into the area or volume between the filter 50 and the catheter 10. Accordingly, soft tissue or other tissue or particles may not grow into or pass into the through bores 30. Thus, the through bores 30 may remain substantially open and clear for draining of the CSF, or the fluid 60, for an extended period of time.

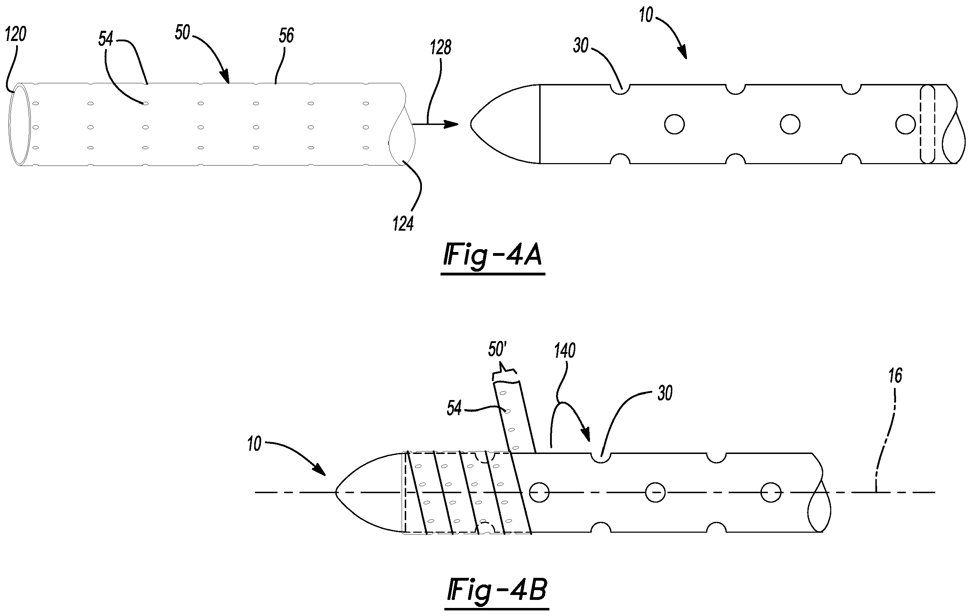

With continuing reference to Figs. 1 and 2, and additional reference to Fig. 4A, the filter 50 may be formed as a tube having the outer wall 56 and a first terminal end 120 and a second terminal end 124. The filter 50, formed as the tube and may then be moved in the direction of arrow 128 to be positioned (i.e. assembled) over the catheter 10, as illustrated in Fig. 2.

The filter 50 may be fixed to the catheter 10 in an appropriate manner, such as by an appropriate fixation member, adhesive, melting, welding, or the like. For example, an adhesive material may be positioned near the second terminal end 124 and near the first terminal end 120 as first and second respective adhesive bands or portions 130, 134. It is understood that the adhesive bands or portions 130, 134 may be applied as a liquid, a solid, or the like and may be provided to substantially surround the catheter 10. Thus, the adhesive portions 130, 134 may seal the respective ends, such as near the terminal ends 120, 124, of the filter 50 relative to the inlet area 20. In various embodiments, the inlet area 20 is substantially and/or completely sealed relative to the external environment 40 save for the through bores 30 of the filter 50.

In various embodiments, returning reference to Fig. 1B, a selected number of the bores 30 may be left uncovered by the filter 50. For example, the bores 30 may be formed in a plurality of rows 30a, 30b, etc. spaced apart axially along the catheter 10. A selected number of holes 30, such as a selected number of rows (e.g. one or two rows) 30a may be left open and/or uncovered by the filter 50. In other words, the filter may end at end 50a illustrated in phantom. Thus, one skilled in the art will understand that the filter will not need or be required to cover all of the holes 30.

In various embodiments, the filter may be provided as a filter strip or portion 50' that may be wrapped onto the catheter 10 to form a filter construct on the catheter 10. As illustrated in Fig. 4B, the filter material 50' may be wrapped around the catheter 10. For wrapping, the filter material 50' may be formed or provided as an elongated member. The filter material 50' may then be wrapped generally around the long axis 16 of the catheter 10. In various embodiments, the filter material 50' may be wrapped in an annular or circular motion, such as generally in the direction of arrow 140 around the catheter 10. The filter material 50' may be wrapped to ensure overlap and/or contact of the edges such that the filter material forms a complete covering over the passages 30. In various embodiments, the edges of the wrap may further include an adhesive to ensure a seal. The filter material 50' may therefore be formed onto or positioned onto the catheter 10 to form the filter 50 on the catheter 10. Again the filter 50 may be fixed to the catheter 10, such as over the inlet area 20, with an appropriate adhesive, physical connection, or the like.

In light of the above, the inlet catheter 10 is provided with the filter 50 to minimize or eliminate passage or contact of material with the catheter save for the liquid 60. As discussed above, the liquid 60 may include CSF that surrounds or is in the external environment 40 relative to the catheter 10. Thus, the CSF may flow through the filter 50 into the internal passage 38 of the catheter 10, and be shunted away to an appropriate location, such as a peritoneal cavity of the subject 84. The filter 50, therefore, may protect and eliminate in-growth or contact of non-liquid material with the catheter 10, particularly in the inlet area 20, and/or only in the inlet area 20, for an extend use of the catheter 10. In various embodiments, the filter material may operate as a protector to inhibit, including to eliminate, ingrowth and/or encapsulation of the catheter by material, such as tissue ingrowth.

Example embodiments are provided so that this disclosure will be thorough, and will fully convey the scope to those who are skilled in the art. Numerous specific details are set forth such as examples of specific components, devices, and methods, to provide a thorough understanding of embodiments of the present disclosure. It will be apparent to those skilled in the art that specific details need not be employed, that example embodiments may be embodied in many different forms and that neither should be construed to limit the scope of the disclosure. In some example embodiments, well-known processes, well-known device structures, and well-known technologies are not described in detail.

The foregoing description of the embodiments has been provided for purposes of illustration and description. It is not intended to be exhaustive or to limit the disclosure. Individual elements or features of a particular embodiment are generally not limited to that particular embodiment, but, where applicable, are interchangeable and can be used in a selected embodiment, even if not specifically shown or described. The same may also be varied in many ways. Such variations are not to be regarded as a departure from the disclosure, and all such modifications are intended to be included within the scope of the disclosure. The scope of protection of the current invention is defined by the appended claims.

## Claims

1. A hydrocephalus shunt system, comprising:
a catheter (10) including an inlet portion (20) having length between a first end and a second end, wherein the inlet portion (20) includes an external wall (12) defining a cannula (38) and an external wall surface (14), a first through passage (30) extending from the external wall surface (14) to the cannula (38), wherein the first through passage (30) defines a first internal dimension;
an outlet portion extending away from the second end;
a filter member (50) formed as a tube having an outer wall (56) and a first terminal end (120) and a second terminal end (124), the filter member (50) configured to extend over the inlet portion (20) and cover the first through passage (30), wherein the filter member (50) includes a second through passage (54), wherein the second through passage (54) defines a second internal dimension less than the first internal dimension; and
first and second adhesive bands (130, 134) respectively positioned near the second terminal end (124) and the first terminal end (120) and fixing the filter member (50) to the catheter (10), the first and second adhesive bands (130, 134) surrounding the catheter (10), and the first and second adhesive bands (130, 134) sealing the filter member (50) at the first terminal end (120) and the second terminal end (124) relative to the inlet portion (20),
wherein a fluid (60) is operable to flow through the second through passage (54), between the filter member (50) and the external wall surface (14) of the inlet portion (20), into the inlet portion (20) through the first through passage (30) and to the outlet portion.

2. The system of Claim 1, further comprising:
a flow regulating portion (88) between the inlet portion (20) and the outlet portion.

3. The system of any one of Claims 1 or 2, wherein the filter member (50) includes an external wall (56) having an external surface and an internal surface, wherein the second through passage (54) extends from the external surface to the internal surface.

4. The system of Claim 3, wherein the second through passage (54) includes a plurality of second through passages (54) formed along a length of the filter member (50).

5. The system of claim 4, wherein each of the plurality of second through passages (54) are configured to allow a flow of a fluid (60) there through while inhibiting impedance of the flow of the fluid (60) due to a solid.

6. The system of Claim 4, wherein the plurality of second through passages (54) are formed separately from formation of the filter member (50).

7. The system of any one of Claims 1 to 6, wherein the filter member (50) is formed of a material including polytetrafluoroethylene.

8. A method of assembling a hydrocephalus shunt system of Claim 1, comprising:
assembling the filter member (50) onto the inlet portion (20); and
preparing the assembly of the filter member (50) and the inlet portion (20).

9. The system of any one of Claims 1 to 7, wherein the second internal dimension allows passage of a fluid (60) and inhibits ingrowth of a tissue.

## Patentansprüche

1. Hydrocephalus-Shunt-System, umfassend:
einen Katheter (10), der einen Einlassabschnitt (20) mit einer Länge zwischen einem ersten Ende und einem zweiten Ende umfasst, wobei der Einlassabschnitt (20) eine Außenwand (12), die eine Kanüle (38) und eine Außenwandoberfläche (14) definiert, eine erste Durchgangspassage (30), die sich von der Außenwandoberfläche (14) zu der Kanüle (38) erstreckt, einschließt, wobei die erste Durchgangspassage (30) eine erste Innenabmessung definiert;
einen Auslassabschnitt, der sich von dem zweiten Ende weg erstreckt;
ein Filterelement (50), das als Schlauch mit einer Außenwand (56) und einem ersten Endabschnitt (120) und einem zweiten Endabschnitt (124) ausgebildet ist, wobei das Filterelement (50) konfiguriert ist, um sich über den Einlassabschnitt (20) zu erstrecken und die erste Durchgangspassage (30) zu bedecken, wobei das Filterelement (50) eine zweite Durchgangspassage (54) einschließt, wobei die zweite Durchgangspassage (54) eine zweite Innenabmessung definiert, die kleiner ist als die erste Innenabmessung; und
erste und zweite Klebebänder (130, 134), die jeweils nahe dem zweiten Endabschnitt (124) und dem ersten Endabschnitt (120) positioniert sind und das Filterelement (50) an dem Katheter (10) befestigen, wobei die ersten und zweiten Klebebänder (130, 134) den Katheter (10) umgeben, und die ersten und zweiten Klebebänder (130, 134) das Filterelement (50) an dem ersten Endabschnitt (120) und dem zweiten Endabschnitt (124) relativ zu dem Einlassabschnitt (20) abdichten,
wobei ein Fluid (60) geeignet ist, um durch die zweite Durchgangspassage (54), zwischen dem Filterelement (50) und der Außenwandoberfläche (14) des Einlassabschnitts (20), in den Einlassabschnitt (20) durch die erste Durchgangspassage (30) und zu dem Auslassabschnitt zu fließen.

2. System nach Anspruch 1, ferner umfassend:
einen Strömungsregulierungsabschnitt (88) zwischen dem Einlassabschnitt (20) und dem Auslassabschnitt.

3. System nach einem der Ansprüche 1 oder 2, wobei das Filterelement (50) eine Außenwand (56) mit einer Außenoberfläche und einer Innenoberfläche einschließt, wobei sich die zweite Durchgangspassage (54) von der Außenoberfläche zu der Innenoberfläche erstreckt.

4. System nach Anspruch 3, wobei die zweite Durchgangspassage (54) eine Vielzahl von zweiten Durchgangspassagen (54) einschließt, die entlang einer Länge des Filterelements (50) ausgebildet sind.

5. System nach Anspruch 4, wobei jede der Vielzahl von zweiten Durchgangspassagen (54) konfiguriert ist, um eine Strömung eines Fluids (60) dort hindurch zu ermöglichen, während ein Strömungswiderstand des Fluids (60) aufgrund eines Feststoffs gehemmt wird.

6. System nach Anspruch 4, wobei die Vielzahl von zweiten Durchgangspassagen (54) getrennt von der Bildung des Filterelements (50) ausgebildet werden.

7. System nach einem der Ansprüche 1 bis 6, wobei das Filterelement (50) aus Material ausgebildet ist, das Polytetrafluorethylen einschließt.

8. Verfahren zum Zusammenbauen eines Hydrocephalus-Shunt-Systems nach Anspruch 1, umfassend:
Zusammenbauen des Filterelements (50) auf dem Einlassabschnitt (20); und
Vorbereiten des Zusammenbauens des Filterelements (50) und des Einlassabschnitts (20).

9. System nach einem der Ansprüche 1 bis 7, wobei die zweite Innenabmessung die Passage eines Fluids (60) ermöglicht und das Einwachsen eines Gewebes hemmt.

## Revendications

1. Système de dérivation d'hydrocéphalie, comprenant :
un cathéter (10) comportant une partie d'entrée (20) ayant une longueur entre une première extrémité et une seconde extrémité, dans lequel la partie d'entrée (20) comporte une paroi externe (12) définissant une canule (38) et une surface de paroi externe (14), un premier passage traversant (30) s'étendant à partir de la surface de paroi externe (14) vers la canule (38), dans lequel le premier passage traversant (30) définit une première dimension interne ;
une partie de sortie s'étendant à l'écart de la seconde extrémité ;
un élément filtrant (50) formé en tant que tube ayant une paroi externe (56) et une première extrémité terminale (120) et une seconde extrémité terminale (124), l'élément filtrant (50) étant conçu pour s'étendre par-dessus la partie d'entrée (20) et couvrir le premier passage traversant (30), dans lequel l'élément filtrant (50) comporte un second passage traversant (54), dans lequel le second passage traversant (54) définit une seconde dimension interne inférieure à la première dimension interne ; et
des première et seconde bandes adhésives (130, 134) positionnées respectivement près de la seconde extrémité terminale (124) et de la première extrémité terminale (120) et fixant l'élément filtrant (50) au cathéter (10), les première et seconde bandes adhésives (130, 134) entourant le cathéter (10), et les première et seconde bandes adhésives (130, 134) scellant l'élément filtrant (50) au niveau du premier une extrémité terminale (120) et la seconde extrémité terminale (124) par rapport à la partie d'entrée (20),
dans lequel un fluide (60) peut s'écouler à travers le second passage traversant (54), entre l'élément filtrant (50) et la surface de paroi externe (14) de la partie d'entrée (20), dans la partie d'entrée (20) à travers le premier passage traversant (30) et vers la partie de sortie.

2. Système selon la revendication 1, comprenant en outre :
une partie de régulation de débit (88) entre la partie d'entrée (20) et la partie de sortie.

3. Système selon l'une quelconque des revendications 1 ou 2, dans lequel l'élément filtrant (50) comporte une paroi externe (56) ayant une surface externe et une surface interne, dans lequel le second passage traversant (54) s'étend de la surface externe à la surface interne.

4. Système selon la revendication 3, dans lequel le second passage traversant (54) comporte une pluralité de seconds passages traversants (54) formés sur une longueur de l'élément filtrant (50).

5. Système selon la revendication 4, dans lequel chacun de la pluralité de seconds passages traversants (54) est conçu pour permettre l'écoulement d'un fluide (60) à travers celui-ci tout en inhibant l'impédance de l'écoulement du fluide (60) dû à un solide.

6. Système selon la revendication 4, dans lequel la pluralité de seconds passages traversants (54) sont formés séparément de la formation de l'élément filtrant (50).

7. Système selon l'une quelconque des revendications 1 à 6, dans lequel le premier élément (50) est formé d'un matériau comprenant du polytétrafluoroéthylène.

8. Procédé d'assemblage d'un système de dérivation d'hydrocéphalie selon la revendication 1, comprenant :
l'assemblage de l'élément filtre (50) sur la partie d'entrée (20) ; et
la préparation de l'ensemble de l'élément filtre (50) et de la partie d'entrée (20).

9. Système selon l'une quelconque des revendications 1 à 7, dans lequel la seconde dimension interne permet le passage d'un fluide (60) et inhibe la croissance d'un tissu.
